# FASCICULE DE BREVET EUROPEEN

(11) **EP 0 757 722 B1**
(45) Date de publication et mention de la délivrance du brevet: **28.06.2006**
(21) Numéro de dépôt: 95917410.3
(22) Date de dépôt: 18.04.1995
(51) Int. Cl.: C12N 15/86, C12N 7/04, C07K 14/055, C12N 15/38, C12N 7/02

(54) **HERPESVIRUS TRANSFORMES POUR EXPRIMER gD IN VITRO**
HERPESVIREN, UM gD IN VITRO ZU EXPRIMIEREN
HERPES VIRUS TRANSFORMED TO EXPRESS gD IN VITRO

(30) Priorité: 20.04.1994 FR 9404975
(43) Date de publication de la demande: 12.02.1997
(73) Titulaire: MERIAL, 69002 Lyon (FR)
(72) Inventeur: AUDONNET, Jean-Christophe Francis, 69006 Lyon (FR); DARTEIL, Raphael Jean, 69007 Lyon (FR); RIVIERE, Michel Albert Emile, 69130 Ecully (FR); ZELNIK, Vladimir, 85 105 Bratislava (SK); ROSS, Louis Joseph Norman, Newbury RG14 6JJ (GB)
(74) Mandataire: Nargolwalla, Cyra
(86) Numéro de dépôt international: PCT/FR1995/000502
(87) Numéro de publication internationale: WO 1995/029248

(56) Documents cités:
- EP-A- 0 431 668
- EP-A- 0 447 303
- EP-A- 0 477 056
- EP-A- 0 496 135
- EP-A- 0 510 996
- WO-A-90/02803
- WO-A-92/03547
- WO-A-93/25665
- US-A- 5 231 023

## Description

La présente invention a trait à de nouveaux herpèsvirus recombinés obtenus à partir d'herpèsvirus naturellement déficients en gD, notamment herpèsvirus aviaires et alphaherpèsvirus, en particulier alphaherpèsvirus aviaires et plus particulièrement MDV sérotypes 1, 2 et 3, ainsi que notamment VZV. Elle a également trait entre autres à leur utilisation comme vaccins et à leur procédé de préparation.

Le virus responsable de la maladie de Marek (Marek's disease virus ou MDV) chez le poulet est un alphaherpèsvirus. Trois sérotypes du virus MDV sont décrits (Bülow V.V. et Biggs P.M., Avian Pathology, 1975, 4, 133-146). Seuls les virus du sérotype 1 oncogènes sont pathogènes chez le poulet. Leur réplication chez l'hôte provoque la formation de lymphomes des cellules T et une démyélination des nerfs périphériques moteurs se traduisant en général par des signes de parésie ou de paralysie chez les animaux atteints (Calnek et Witter, Marek's Disease in Diseases of Poultry 9th édition, pp 342-390, Calnek B.W. et al. Ed. 1991, Iowa State Univ. Press). Cette maladie, aux conséquences économiques très importantes pour l'élevage aviaire, est prévenue par une vaccination des poussins d'un jour avec soit l'herpèsvirus du dindon (Herpesvirus of turkey ou HVT ou encore MDV de sérotype 3), virus antigéniquement proche du virus MDV mais non pathogène chez le poulet, soit des souches vivantes MDV non oncogènes et non pathogènes chez le poulet, appartenant aux sérotypes 1 ou 2.

Pour les besoins de la production de vaccins, ces trois sérotypes sont principalement cultivés sur cellules primaires d'embryons de poulets (CEPs). Les cellules primaires d'embryons de canards peuvent également être utilisées pour multiplier les virus MDV de sérotype 2. Lors de la culture in vitro, ces virus sont fortement associés aux cellules. Ce phénomène est connu depuis longtemps (Churchill A.E. et Biggs P.M. Nature, 1967, 215, 528-530), et il a été également constaté que les virions produits après culture sur CEPs étaient non infectieux par voie orale ou nasale pour les poulets. La vaccination avec ces virus nécessite donc leur administration par voie parentérale, en général par transfixion de la membrane alaire ou par injection intramusculaire, ce qui augmente de façon non négligeable le coût de la vaccination contre la maladie de Marek.

Il existe par contre chez le poulet une production de virus MDV infectieux, c'est-à-dire transmissible par voie aérienne ou par simple contact, au cours de l'infection par les souches pathogènes du virus MDV. Cette production de virus infectieux se fait au niveau des follicules plumeux, et le virus est disséminé dans l'environnement par l'intermédiaire des plumes de mue, des squames et des poussières (Calnek B.W. et al., Avian Diseases, 1970, 14, 219-233). C'est par ce mécanisme que s'effectue la diffusion naturelle de la maladie dans les élevages. Les différences observées entre la culture in vitro et la réplication in vivo du virus MDV n'ont jamais été expliquées jusqu'à ce jour.

L'étude de la culture in vitro des virus HVT ou MDV sur CEPs a montré que ces virus étaient étroitement associés aux membranes cellulaires. Cela a pour conséquence la quasi absence de virions libres et infectieux dans les surnageants de cultures.

Il vient d'être montré récemment qu'un gène codant pour une glycoprotéine homologue de HSV-1 gD est présent sur les génomes respectifs des virus HVT et MDV (Ross L.J.N. et al., J. Gen. Virol. 1991, 72, 949-954 ; Zelnik V. et al., J. Gen. Virol. 1993, 74, 2151-2162). Les produits d'expression de ces gènes chez HVT et MDV ont été étudiés et recherchés dans les lysats de CEPs infectées par ces virus. Ces expériences n'ont toutefois pas permis de démontrer l'expression de la glycoprotéine gD chez ces deux virus.

En effet, des antigènes MDV gD exprimés sous forme de protéines de fusion avec la protéine TrpE chez E. coli ont été utilisés pour induire la production d'anticorps chez des lapins et, curieusement, les sérums ainsi préparés n'ont pas reconnu de protéine spécifique dans les lysats de CEPs infectées avec HVT ou MDV, alors que les anticorps préparés à partir de protéines de fusion exprimant des antigènes MDV gI ou MDV gE reconnaissent les glycoprotéines correspondantes exprimées par les virus MDV ou HVT au cours de la culture in vitro (Brunovskis P. et al, Proceedings 19th World's Poultry Congress, pp118-122. Amsterdam, 19-24 Septembre 1992). Cela indique soit une absence d'expression, soit un très faible niveau d'expression de gD au cours de la culture in vitro de HVT et MDV.

A l'inverse, des sérums de poulets infectés par les souches pathogènes de MDV reconnaissent les antigènes HVT gD ou MDV gD exprimés sous forme de protéines de fusion avec la glutathion-transférase (Zelnik V. et al., Proceedings 19th World's Poultry Congress, pp 114-117. Amsterdam, 19-24 Septembre 1992). Ces résultats montrent que les gènes HVT gD et MDV gD sont bien exprimés au cours de la réplication in vivo chez le poulet et que les glycoprotéines respectives sont non seulement immunogènes, mais partagent aussi certains déterminants antigéniques. Il est à noter que chez la plupart des alphaherpèsvirus étudiés à ce jour, la glycoprotéine gD est un immunogène majeur qui contribue de façon significative à l'induction de l'immunité protectrice antivirale chez l'animal infecté ou immunisé.

X. Tan et L.F. Velicer (Abstracts of the XVIII International Herpesvirus Workshop, University of Pittsburgh Médical Center, Pittsburgh, PA, 1993, p.A145) ont aussi déclaré que gD n'était pas exprimé en culture cellulaire, ou tout au moins pas à un niveau détectable par Northern blot, ce qui pourrait expliquer la nature cellule-associée du virus MDV. Ces auteurs proposent que MDV gD est exprimé dans l'épithélium des follicules plumeux de poulet où MDV est formé sous forme de virions infectieux enveloppés non associés aux cellules. Ceci est également montré dans la demande internationale WO 92/03587

La présente invention est basée sur la découverte que la mise en oeuvre d'une expression constitutive de la glycoprotéine gD par le virus MDV, y compris HVT, permet de manière surprenante d'obtenir, comme lors de l'infection chez le poulet, des virions libres non associés aux cellules. Cette particularité offre la possibilité très avantageuse d'administrer ce virus par voie aérienne à des fins vaccinales et permet par ailleurs d'obtenir du virus libre dans le surnageant des cultures in vitro, ce qui facilite l'obtention et la fabrication du vaccin contre la maladie de Marek.

De manière surprenante, la présente invention permet, par remplacement du promoteur naturel du gène gD chez les virus HVT et MDV par un autre promoteur, l'expression in vitro des glycoprotéines HVT gD et MDV gD. L'expression de gD, obtenue notamment en plaçant ce gène sous le contrôle d'un promoteur non régulé par le virus, permet la maturation complète des virions qui peuvent alors "sortir" librement des cellules infectées. Cette expression de gD in vitro est réalisée en remplaçant le promoteur naturel du gène gD par un autre promoteur qui peut être notamment choisi parmi les promoteurs eucaryotes forts classiquement connus (par exemple promoteurs HCMV IE, SV40, ou promoteurs précoces endogènes des virus HVT ou MDV...) ou promoteurs tardifs (par exemple promoteurs tardifs de MDV ou HVT, notamment de gB MDV ou HVT,...). Cette expression nouvelle peut être obtenue en utilisant les moyens offerts par les techniques de recombinaison génétique, en remplaçant par exemple la région située en amont de l'ATG du gène gD par un promoteur exogène de type HCMV IE ou SV40, ou en transposant le gène gD dans la région unique longue du virus et en le plaçant sous le contrôle d'un promoteur endogène ou exogène. De nombreuses possibilités sont ainsi réalisables pour l'expression du gène gD en combinant différents promoteurs et différents sites d'insertion potentiels (tels que les sites RR2, TK, gD, gI, US3, US2, US10...).

Cette expression a des conséquences nouvelles et inattendues sur les propriétés biologiques du virus recombinant. L'expression de la glycoprotéine gD au cours de la réplication in vitro des virus recombinants HVT et MDV ainsi construits se traduit en effet par l'incorporation , de la glycoprotéine gD dans l'enveloppe. virale et par l'obtention de virions infectieux à un titre élevé dans les surnageants de culture. Par ailleurs, les virus recombinants ainsi obtenus, tout en gardant les caractères d'atténuation des virus parentaux, sont plus infectieux pour les lymphocytes in vivo et se répliquent plus rapidement dans ces cellules. Cela a pour conséquence directe une plus grande et une plus rapide diffusion de ces virus au sein de l'organisme du poulet. La protection contre la maladie de Marek est donc meilleure, quelle que soit la voie d'administration (oronasale ou parentérale). Du fait de ces modifications biologiques dans la réplication du virus, la protection contre la maladie de Marek peut être obtenue plus précocement et avec des doses vaccinales beaucoup plus réduites qu'avec les vaccins classiquement utilisés.

On a en outre découvert qu'il était possible d'étendre cet enseignement aux autres herpèsvirus dont la structure génomique et les propriétés biologiques in vitro et in vivo sont équivalentes à celles des herpèsvirus aviaires mentionnés ci-dessus. Par exemple, la présente invention permet l'obtention d'un virus VZV recombiné exprimant le gène gD d'un autre virus tel que HSV-1 ou HSV-2, avec pour conséquence très avantageuse la production de virus VZV libre par culture in vitro sur cellules. Ce virus recombiné peut constituer un vaccin contre VZV et contre le virus dont est issu le gène gD, par exemple HSV-1 ou HSV-2.

Il existe en fait deux grands types de virus concernés par l'invention, qui correspondent à la définition de virus déficient en gD, à savoir ceux qui n'expriment pas gD in vitro en cultures cellulaires, ce qui est le cas des virus MDV, y compris HVT, et ceux qui sont dépourvus d'un gène homologue du gène gD, ce qui est le cas du virus VZV (varicella-zoster virus).

L'invention concerne donc tous les herpèsvirus équivalents, dans le sens qui vient d'être défini, aux virus-types MDV et VZV.

La présente invention a donc pour objet un herpèsvirus naturellement déficient en gD (dans le sens de l'invention défini ci-dessus), transformé pour exprimer une glycoprotéine gD au cours de sa réplication in vitro. De préférence, il s'agit d'un herpèsvirus aviaire, notamment virus MDV des sérotypes 1, 2 et 3 ou bien du virus de la varicelle VZV.

Selon un premier mode de réalisation préféré de l'invention, qui concerne les herpèsvirus ayant un gène gD non exprimé in vitro, l'herpèsvirus a son gène gD placé sous le contrôle d'un promoteur autre que le promoteur naturel de ce gène. Le promoteur naturel du gène gD est remplacé par un promoteur endogène (c'est-à-dire un promoteur de ce virus ou des autres sérotypes de ce virus, notamment un promoteur précoce) ou exogène, permettant l'expression du gène gD in vitro.

Dans un deuxième mode de réalisation préféré de l'invention, l'herpèsvirus comprend une cassette d'expression comportant le gène gD placé sous le contrôle d'un promoteur permettant son expression lors de la réplication du virus in vitro.

Dans le cas d'un herpèsvirus ne présentant pas naturellement de gène gD, la cassette comprend le gène gD d'un autre virus, par exemple de HSV ou même d'un virus tel que MDV, y compris HVT, et un promoteur approprié qui peut être le promoteur naturel du gène inséré ou être un autre promoteur, ce qui sera le cas lorsque le gène gD proviendra d'un virus du type MDV, la condition étant d'obtenir une expression du gène gD in vitro.

Dans le cas d'un herpèsvirus seulement déficient dans l'expression de gD in vitro, la cassette peut aussi comprendre aussi bien le gène gD endogène qu'un autre gène gD, et un promoteur approprié comme défini ci-dessus.

La cassette d'expression est insérée dans un site non essentiel pour la réplication du virus et pour l'efficacité du virus à titre de vaccin. Pour les virus MDV des sérotypes 1, 2 et 3, un site d'insertion est avantageusement choisi parmi le groupe consistant en: US2, US3, US10, gI, gD, TK, RR2, UL13. On comprend bien entendu que cette énumération n'est pas limitative et que tout site d'insertion potentiel peut être utilisé. Pour le virus VZV, on peut choisir un site d'insertion parmi le groupe consistant en: TK, US3, gI, gE, gC, région intergénique US3-gI.

On comprend que le gène gD, au sein d'une cassette telle que définie ci-dessus et quelle que soit son origine, peut être inséré dans MDV, ou autre virus du même type, à la place du gène gD lui-même ou dans un autre site d'insertion.

La présente invention a de préférence pour objet un herpèsvirus choisi parmi le groupe consistant en: MDV des sérotypes 1, 2 et 3 et VZV, exprimant une glycoprotéine gD au cours de sa réplication in vitro.

La présente invention a pour objet la production in vitro de virus, notamment HVT et MDV, infectieux, sous forme de virions libres présents dans le surnageant de culture de cellules. Les techniques de culture sont notamment celles habituellement utilisées dans la pratique pour un virus donné. Celles-ci sont bien connues de l'homme du métier et n'ont donc pas à être décrites plus en détail ici. L'utilisation des virus transformés conformément à l'invention dans la production de virus facilite la fabrication de vaccins, notamment ceux contre la maladie de Marek, et de virus recombinants, notamment HVT et MDV, en permettant d'obtenir un effet cytopathique généralisé plus rapide (2 à 3 jours selon la richesse de l'inoculum) et une meilleure résistance, du virus aux opérations de conservation par congélation ou par lyophilisation. On obtient avantageusement des titres élevés en virus libres dans le surnageant de culture en comparaison de ce qui est obtenu en l'absence d'expression de gD. Il s'agit là d'un résultat remarquable.

L'invention a donc pour objet un procédé de culture d'herpèsvirus naturellement déficient en gD, dans lequel on cultive sur cellules le virus qui a été transformé par recombinaison génétique pour exprimer la glycoprotéine gD, puis l'on recueille les virions produits dans le surnageant de culture. Les techniques de culture des virus sont notamment celles habituellement utilisées dans la pratique. L'homme du métier est parfaitement au fait des techniques appropriées à chaque virus et celles-ci n'ont donc pas à être décrites plus en détail ici.

L'invention a également pour objet les cultures d'herpèsvirus susceptibles d'être obtenues par ce procédé. Ces cultures se caractérisent notamment par le fait qu'elles comprennent le virus, notamment MDV, HVT, VZV, à l'état de virion libre, c'est-à-dire non associé à des cellules, et présentent les propriétés et qualités déjà indiquées.

La présente invention a aussi pour objet la production in vitro de virus, notamment HVT ou MDV, infectieux qui peuvent être utilisés comme vecteurs d'expression pour des gènes étrangers, en particulier pour des gènes ou des fractions de gènes codant pour des antigènes induisant une protection contre des maladies, en particulier une protection des oiseaux d'élevage contre des virus, bactéries ou parasites pathogènes responsables de maladies aviaires, et plus particulièrement des poulets.

L'invention a donc également pour objet les herpèsvirus tels que définis plus haut, qui comprennent en outre au moins un gène hétérologue inséré dans leur génome de manière que ce gène puisse s'exprimer in vivo. Pour cela, le virus recombiné peut comprendre soit une double cassette incluant le gène gD, le promoteur de celui-ci et le gène codant pour l'antigène avec un promoteur approprié, la cassette pouvant être insérée dans le site gD pour un virus tel que MDV, ou dans un autre site, soit une cassette pour chaque gène, celle comprenant le gène gD pouvant être insérée par exemple dans un site autre que le site gD naturel dans le cas d'un virus tel que MDV et l'autre cassette pouvant être insérée par exemple dans le site gD naturel, ou dans tout autre site approprié, soit encore seul le promoteur naturel du gène gD est remplacé et la cassette comprenant le gène hétérologue est insérée dans un autre site approprié.

Il est remarquable de noter que lorsque le virus tel que MDV a été transformé pour exprimer un gène gD endogène ou d'un autre sérotype sous le contrôle d'un promoteur efficace selon l'invention, ce gène inséré est exprimé in vivo au cours de la virémie et pas seulement dans les follicules plumeux (cas du gD naturel), ce qui se traduit notamment par une réponse immunitaire, en particulier cellulaire, plus forte et plus précoce.

La présente invention a encore pour objet la production de vaccins avantageusement utilisables par les voies aérienne (nasale, oculaire), orale ou parentérale, notamment pour immuniser les poulets contre la maladie de Marek et les autres maladies correspondant aux antigènes spécifiques exprimés par les virus HVT ou MDV recombinants.

Les vaccins selon l'invention se caractérisent par le fait qu'ils comprennent un virus vivant tel que défini plus haut et qu'ils peuvent se présenter notamment sous la forme lyophilisée ou congelée.

Le procédé de production d'un vaccin vivant selon l'invention se caractérise par le fait que l'on cultive sur cellules un herpèsvirus naturellement déficient en gD, qui a été transformé pour exprimer gD lors de sa réplication in vitro et éventuellement transformé pour exprimer in vivo au moins un gène hétérologue codant pour un antigène ou immunogène vaccinal.

La présente invention a également pour objet la production de vaccins administrables in ovo pour vacciner les poulets contre la maladie de Marek et les vaccins obtenus.

La présente invention a de manière spécifique pour objet l'utilisation du gène gD comme locus d'insertion pour construire des herpèsvirus aviaires recombinants, notamment HVT ou MDV, exprimant en sus des gènes étrangers d'intérêt vaccinal.

La présente invention a aussi pour objet l'obtention de virus aviaires, notamment HVT ou MDV, infectieux pouvant servir de vecteurs pour l'expression d'antigènes étrangers et permettant la vaccination in ovo.

La présente invention a aussi pour objet une méthode de vaccination notamment des oiseaux d'élevage, utilisant les virus transformés conformément à l'invention et prévoyant notamment leur administration par voie aérienne, bien que les autres voies ne soient pas exclues.

Enfin, la révélation du principe à la base de l'invention permet à l'homme du métier de rechercher aussi chez les virus du type MDV des mutants naturels exprimant gD in vitro. Ces mutants entrent alors dans le cadre de l'invention.

L'invention va être maintenant décrite plus en détail à l'aide d'exemples de réalisation se référant au dessin, dans lequel on voit:
- à la figure 1: par digestion du plasmide pRD001 comprenant un fragment de 29 kpb de l'ADN de HVT, construction des plasmides pRD006 et pRD007 qui comprennent respectivement un fragment de 2,8 kb incluant la partie de gD en 5' de PstI et un fragment de 5,7 kb incluant la partie de gD en 3' de PstI;
- à la figure 2: construction du plasmide pRD015 comprenant la partie flanquante en 5' de gD, à partir du plasmide pRD001;
- à la figure 3: plasmide pRD022 obtenu à partir du vecteur pBS-SK+ et comprenant le gène gD dépourvu de sa partie en 5' du site SacI;
- à la figure 4: plasmide pRD030 comprenant l'entièreté du gène gD et des sites de restriction l'encadrant;
- à la figure 5: à partir du pCMVβ, construction du plasmide pRD031 contenant le gène gD sous le contrôle du promoteur HCMV IE, par remplacement du gène LacZ par gD;
- à la figure 6: plasmide pRD040 comprenant une cassette incluant le gène gD et le promoteur HCMV IE, dans le vecteur pBS-SK+;
- à la figure 7: construction du plasmide pRD041 obtenu par addition, au plasmide pRD040, de la séquence 5' flanquante contenue dans le plasmide pRD015;
- à la figure 8: construction du plasmide pRD043 comprenant le gène gD, le promoteur HCMV, et l'ensemble promoteur SV40 et gène LacZ apporté par pSVβ dans pRD041 entre le promoteur HCMV et la séquence 5' flanquante;
- à la figure 9: construction du plasmide pRD046 à partir du plasmide pBamB contenant un fragment HVT BamHI B incluant le gène TK;

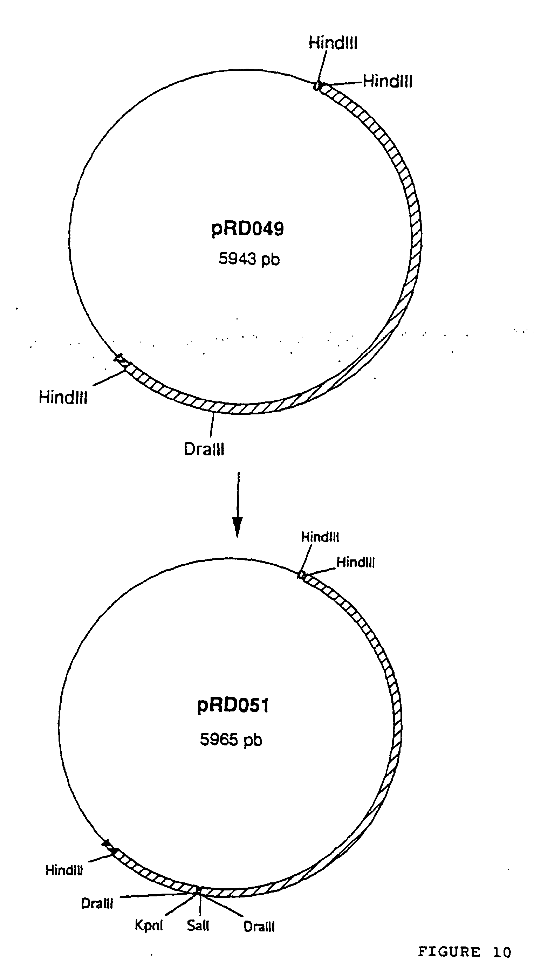

- à la figure 10: construction du plasmide pRD051 par introduction d'un site de restriction multiple dans le site unique DraIII du plasmide pRD049;
- à la figure 11: construction du plasmide pRD053 comprenant la cassette promoteur HCMV et gène HVT-gD insérée dans le gène TK de HVT.

### EXEMPLES

Toutes les techniques utilisées pour les constructions des plasmides donneurs sont les techniques standards de biologie moléculaire décrites par Sambrook et al. (Molecular Cloning : A Laboratory Manual, 2nd Edition, New York, Cold Spring Harbor Laboratory, 1989). Le contenu de cette publication est considéré comme étant incorporé par référence dans cette description.

### Exemple 1 :

Cet exemple avait pour double objectif de démontrer d'une part l'expression de gD dans l'épithélium des follicules plumeux du poulet et d'autre part que, conformément à l'invention, la non expression de gD in vitro est bien liée au promoteur naturel et peut être obtenue par le remplacement du promoteur naturel par un autre promoteur.

### - Plumes et tissus :

Des poussins Rhode Island Red (HPRS RIR) d'un jour ont reçu 1000 u.f.p. (unités formatrices de plaques) d'une souche de MDV RB1B ou HPRS16 et les plumes, bourses, thymus et rates ont été prélevés 4 à 6 semaines après l'inoculation. Les mêmes tissus provenant de poulets SPF non infectés ont servi de témoins.

Les plumes obtenues à partir des régions dorsale et pectorale ont été prises à l'extrémité proximale et les pointes de plume d'environ 0,5 cm de long ont été hachées avec des ciseaux et mises en suspension dans du PBS ou dans un tampon d'extraction (dithiothreitol 5mM, PMSF 50 *µ*g/ml, sarkosyl 1,5 %). En règle générale, on a utilisé 20 pointes de plume par ml de tampon. Après une incubation de 24 à 36 heures à 4°C, les préparations ont été traitées aux ultrasons (3 x 30 s) et centrifugées à 10 000 tr/min dans une microcentrifugeuse. Les surnageants, contenant habituellement 2 µg de protéine par µl, ont été utilisés pour une analyse en Western blot.

Les antigènes ont été extraits des bourses, thymus et rates à l'aide des mêmes tampons que ceux utilisés pour les plumes.

### - CEP infectés par un recombinant vaccine-gD :

Des fibroblastes d'embryon de poulet (CEP) ont été infectés par un recombinant vaccine-gD (virus de la vaccine comportant le gène gD inséré dans un site non essentiel à sa réplication et sous le contrôle d'un promoteur endogène vaccine) à une multiplicité d'infection d'environ 1 u.f.p./cellule et ont été récoltés 3 à 4 jours plus tard lorsque les e.c.p. (effets cytopathiques) étaient très prononcés. Les cellules ont été raclées, rapidement lavées en PBS, et traitées aux ultrasons en PBS à une concentration de 2.10⁷ cellules/ml.

### - Western blot :

Les échantillons destinés à l'analyse en Western blot ont été portés à ébullition dans un tampon dénaturant et 10 µg de plumes ou de tissus ont été appliqués par puits (ou piste). Dans le cas de CEP infectés par la vaccine-gD, seulement 4 µg ont été appliqués par puits. Les polypeptides ont été séparés par électrophorèse sur gel d'acrylamide 12 % en utilisant un appareil mini-gel Biorad et ont été transférés sur nitrocellulose (Hybond C, Amersham). Après transfert, les polypeptides ont été colorés au rouge Ponceau et photographiés pour s'assurer que les quantités de protéine déposées dans chaque puits étaient comparables.

Les blots ont été rincés avec du PBS, bloqués avec 5% de lait écrémé en PBST (25 mM Tris pH 7,6, 0,14 M NaCl et 0,05 % Tween 20) et lavés 4 fois dans du PBST, chaque fois pendant 5 min. Ils ont ensuite été traités avec un surnageant de l'hybridome DA7 (spécifique de gD) dilué au 1/50 dans du PBS contenant 1 % de BSA, pendant 1 h à température ambiante. Après 4 lavages dans du PBST, les blots ont été incubés avec des IgG de chèvre anti-souris conjuguées à la peroxydase (Sigma) diluées au 1/1000 en PBST et les anticorps liés ont été détectés par chimioluminescence (réactif chimioluminescent pour Western blotting Dupont, Dupont). Des pellicules sensibles aux rayons X ont été exposées de 30 secondes à 1 minute selon la technique classique.

### - Test d'immunofluorescence :

Des sections de peau effectuées avec un microtome (Cryostat) ont été fixées à l'acétone pendant 10 min à température ambiante. Elles ont été séchées à l'air, traitées avec 5% de BSA en PBS pendant 30 min à température ambiante pour empêcher les réactions non spécifiques. L'anticorps monoclonal DA7 (dilution au 1/100 en BSA 1% en PBS) ou d'anticorps monoclonal BD1 (liquide d'ascite de souris spécifique de pp38/24 dilué au 1/1000 en BSA 1% en PBS) a ensuite été ajouté pendant 1 h à température ambiante. Les lames ont été lavées 3 fois en PBS, chaque fois pendant 15 min. Des IgG de chèvre anti-souris conjuguées à la FITC, diluées au 1/100 en BSA 1% en PBS, ont ensuite été ajoutées pendant 1 h à température ambiante. Les lames ont été lavées 4 fois en PBS, chaque fois pendant 15 min, plongées dans l'eau, montées dans du glycérol/PBS et examinées sous lumière UV à l'aide d'un microscope à fluorescence.

### - Résultats :

L'analyse Western blot a montré que les préparations de plumes provenant de 3 poulets différents infectés par MDV réagissaient avec l'anticorps monoclonal DA7. L'anticorps a reconnu un polypeptide apparaissant sous forme d'une bande à 55 kDa, ce qui est conforme à la taille attendue pour gD et au produit de traduction in vitro du gène MDV gD (Zelnik et al., J. Gen. Virol. 75, 2747-2753). Des produits de plus faible poids moléculaire (35 kDa et inférieurs) ont été révélés et pourraient correspondre à des formes non glycosylées.

Cela démontre l'expression de gD dans les follicules plumeux chez les poulets infectés par MDV.

Des extraits de poulets non infectés, de CEP non infectés et de CEP infectés par la vaccine seule n'ont pas réagi avec le monoclonal DA7, ce qui démontre la spécificité du test. Les extraits de CEP infectés avec le recombinant vaccine-gD ont donné deux bandes, dont une bande correspondant à la bande 55 kDa de l'essai avec les extraits de plume.

La spécificité du test a encore été contrôlée en démontrant que le monoclonal dirigé contre pp38/24 ne réagissait ni avec les CEP infectés par le recombinant vaccine-gD, ni avec les CEP non infectés. Par contre, ce monoclonal réagit bien comme attendu avec les extraits de plumes de poulets infectés et avec des CEP infectés par MDV.

On a donc démontré qu'à la fois les préparations de plume infectées par MDV et les CEP infectés par le recombinant vaccine-gD réagissaient avec le monoclonal anti-gD et donc que l'expression de ce gène était obtenue in vitro.

Le remplacement du promoteur naturel du gène gD par un autre promoteur permet donc d'exprimer gD in vitro.

### Exemple 2: Construction du plasmide pRD043

La souche FC126 du virus HVT a été isolée en 1968 par le Dr Witter du Regional Poultry Research Laboratory (U.S.D.A., East Lansing, Michigan, U.S.A.), dans un troupeau de dindes de 23 semaines (R.L. Witter et al., Am. J. Vet. Res. 31, 525-538, 1970). Elle a été ensuite passée 10 fois sur des fibroblastes de canard, puis a subi 9 passages supplémentaires sur des fibroblastes d'embryons de poulets EOPS. L'ADN utilisé provient de virus ayant fait l'objet au total de 23 à 24 passages à partir de l'isolat d'origine.

On pourrait aussi utiliser par exemple la souche HVT enregistrée à l'ATCC sous la référence VR584C.

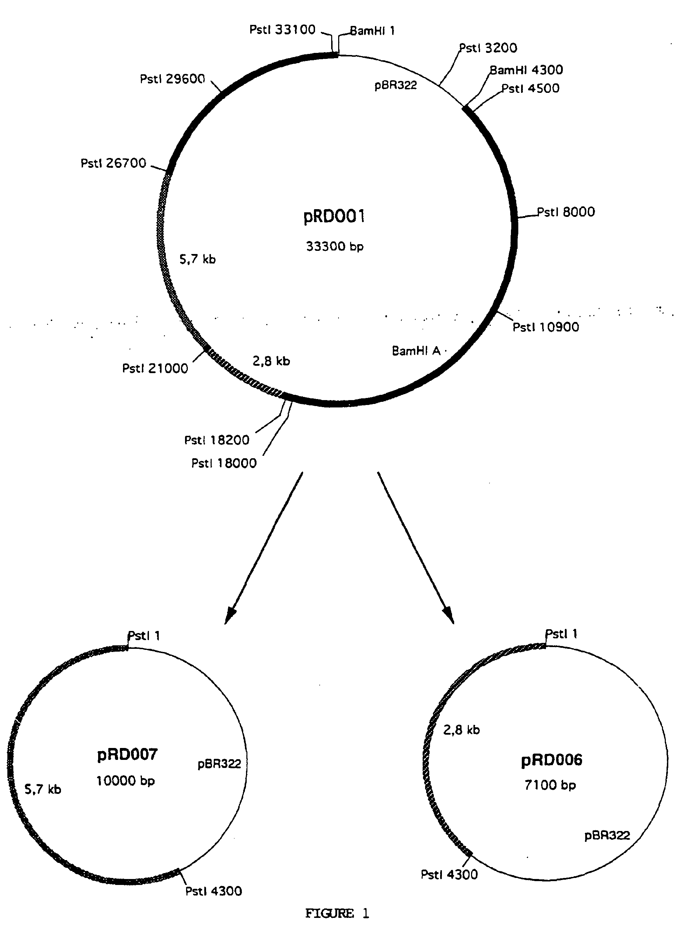

L'ADN génomique du virus HVT a été extrait selon la technique décrite par N. Ross (Ross L.J.N. et al., J. Gen. Virol. 1989, 70, 1789-1804 incorporé ici par référence). Cet ADN a été digéré par BamHI, puis le fragment A de 29 kpb (Igarashi T. et al., Virology 1987, 157, 351-358), comprenant la région Us de HVT, a été cloné dans le vecteur pBR322 préalablement digéré par BamHI pour donner le plasmide pRD001. pRD001 a été digéré par PstI et les fragments PstI-PstI 2,8 kpb et 5,7 kpb ont été clonés dans le plasmide pBR322 digéré par PstI pour donner respectivement les plasmides pRD006 et pRD007 (Figure 1).

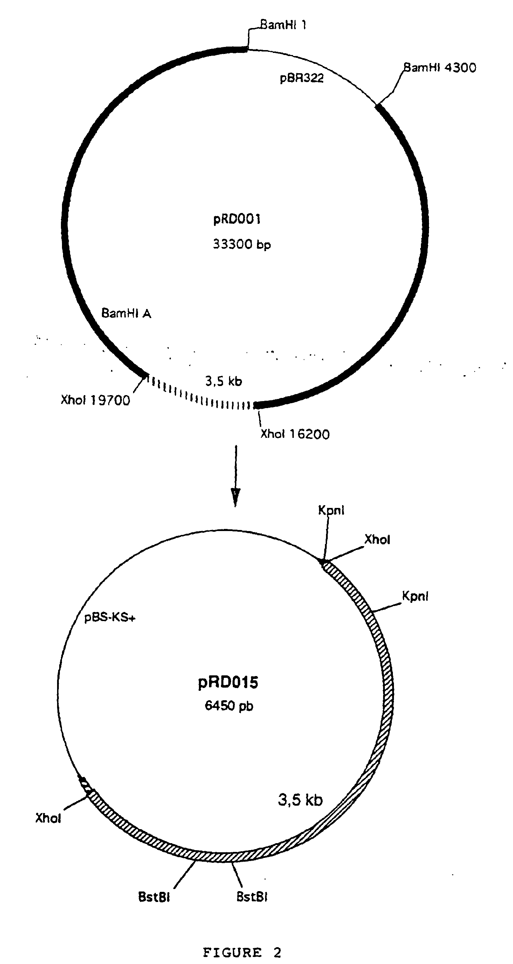

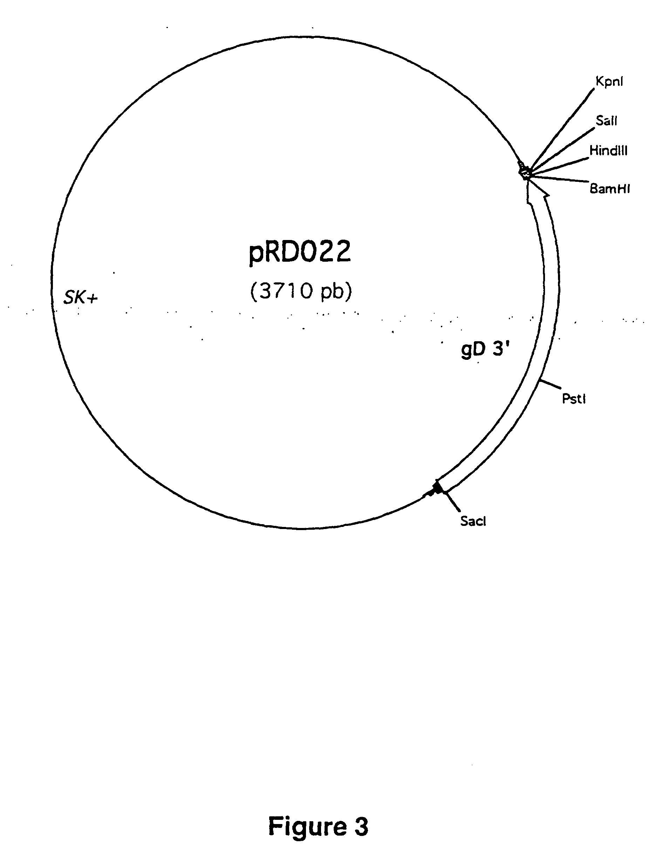

pRD001 a été digéré par XhoI et le fragment XhoI-XhoI 3,5 kpb a été cloné dans le vecteur pBluescript KS+ pour donner le plasmide pRD015 (Figure 2). Le plasmide pRD006 a été digéré par SacI et PstI pour isoler le fragment SacI-PstI de 340 pb (fragment A). Une PCR a été réalisée avec les oligonucléotides RD045 (SEQ ID No. 1 :5' TGCTGGTACCGTCGACAAGCTTGGATCCGTGCAGATAACACGTACTGGC 3') et pBR Pst- (SEQ ID No. 2 : 5' CATGTAACTCGCCTTGATC 3') et la matrice pRD007 pour amplifier la région 3' du gène gD (positions 6491 à 6980 sur la séquence HVT Us (Zelnik V. et al., J. Gen. Virol. 1993, 74, 2151-2162 incorporé ici par référence)). Le fragment PCR de 560 pb a été ensuite digéré par PstI et KpnI pour isoler un fragment B PstI-KpnI de 520 pb. Les fragments A et B ont été clonés dans le vecteur pBS-SK+ préalablement digéré par SacI et KpnI pour donner le plasmide pRD022 (Figure 3), comprenant le gène gD dépourvu d'une partie 5'.

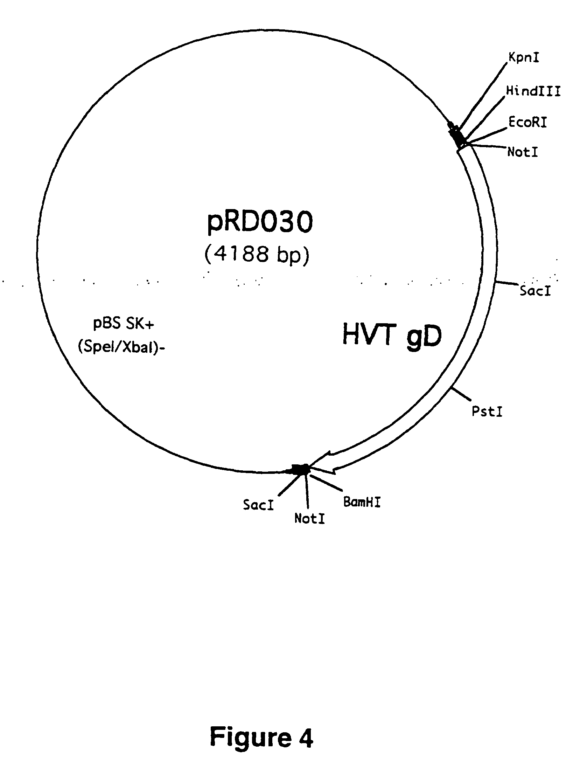

Le plasmide pRD022 a été digéré par BamHI et SacI pour isoler le fragment BamHI-SacI de 850 pb (fragment C). Une PCR a été réalisée avec les oligonucléotides RD050 (SEQ ID No. 3 : 5' TGCTGAATTCGCGGCCGCATGATTATTGTCACCACTTC GAAGATGGC 3') et HVT40M1 (SEQ ID No. 4 : 5' GTCCCCGTTGACAACTACTA 3') et la matrice pRD006 pour amplifier la région 5' du gène gD (positions 5747 à 6286 sur la séquence HVT Us (Zelnik V. et al., J. Gen. Virol. 1993, 74, 2151-2162)). Le fragment PCR de 560 pb a été digéré par EcoRI et SacI pour isoler un fragment D EcoRI-SacI de 420 pb. Les fragments C et D ont été clonés dans un vecteur pBS-SK+ modifié, ne comportant plus les sites XbaI et SpeI (pBS-SK+ digéré par XbaI et SpeI, puis ligaturé), digéré par EcoRI et BamHI pour donner le plasmide pRD030 (Figure 4), comprenant le gène gD.

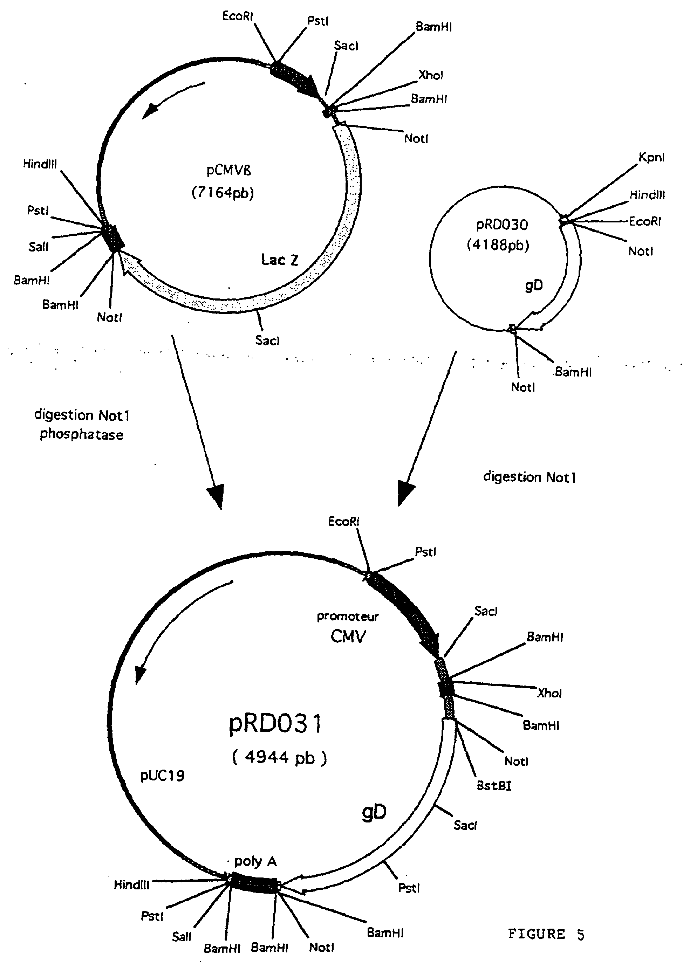

Le plasmide pRD030 a été digéré par NotI pour isoler le fragment NotI-NotI de 1250 pb. Ce fragment a été cloné dans le vecteur pCMVβ (CLONTECH Laboratories, Palo Alto, CA) digéré par NotI pour l'insertion du gène gD à la place de LacZ, ce qui donne le plasmide pRD031 (4944 pb) contenant le gène HVT-gD sous le contrôle du promoteur HCMV IE (Figure 5).

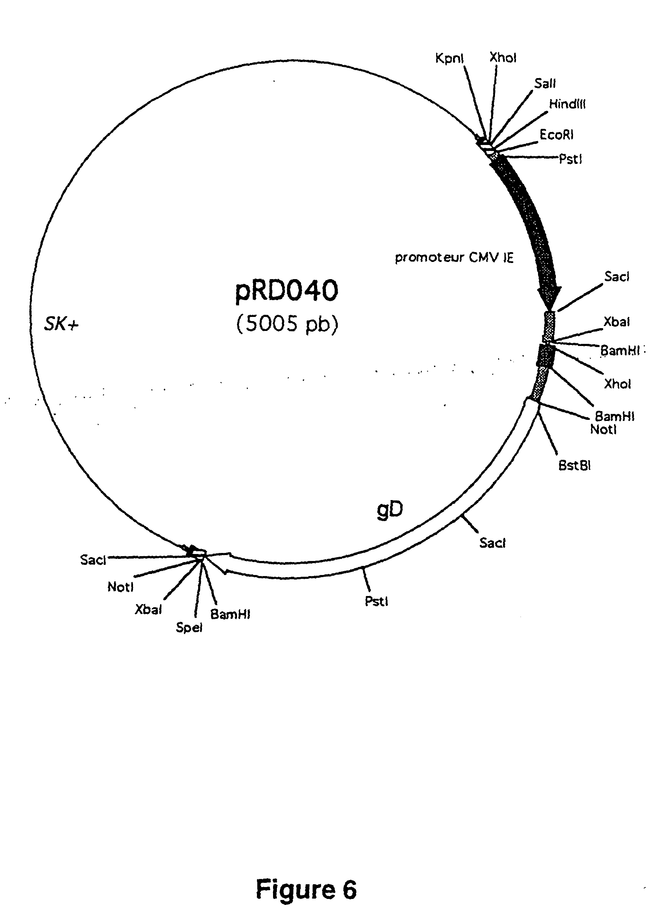

Le plasmide pRD031 a été digéré d'une part par EcoRI et BstBI et d'autre part par BstBI et BamHI pour isoler respectivement le fragment EcoRI-BstBI de 800 pb et le fragment BstBI-BamHI de 1250 pb. Ces deux fragments ont été clonés dans le vecteur pBS-SK+ digéré par EcoRI et BamHI pour donner le plasmide pRD040 (Figure 6) comprenant le promoteur HCMV et gD dans le vecteur SK+.

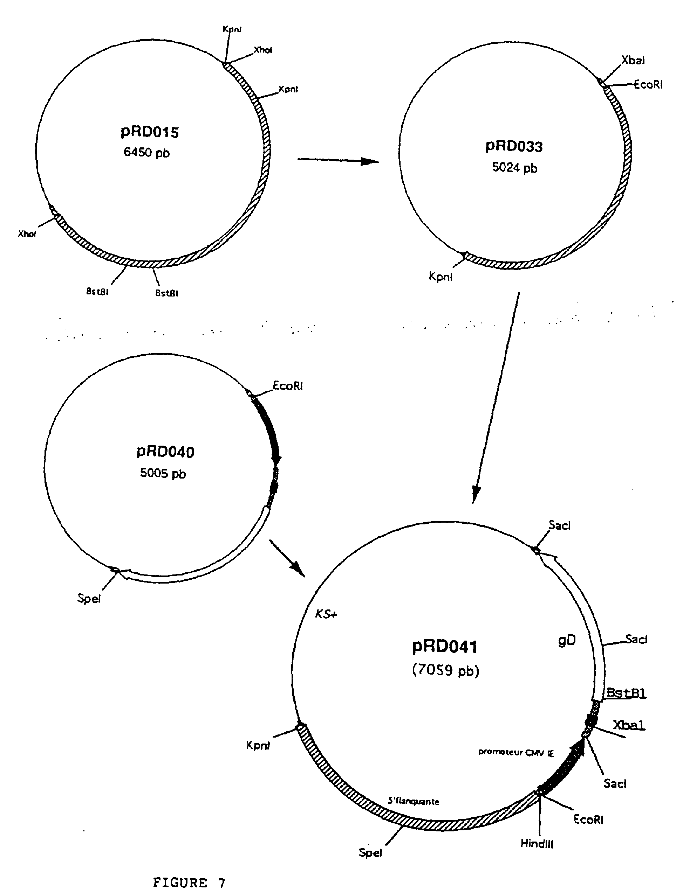

Le plasmide pRD015 a été digéré par KpnI et BstBI pour isoler un fragment KpnI-BstBI de 2100 pb. Ce fragment a été cloné dans le vecteur pBS-KS+ digéré par KpnI et ClaI pour donner le plasmide pRD033 (5024 pb). Le plasmide pRD040 a été digéré par EcoRI et SpeI pour isoler le fragment EcoRI-SpeI de 2000 pb. Ce fragment a été cloné dans le plasmide pRD033 digéré par EcoRI et XbaI pour donner le plasmide pRD041 (Figure 7) comprenant gD, le promoteur HCMV et la région 5' flanquante.

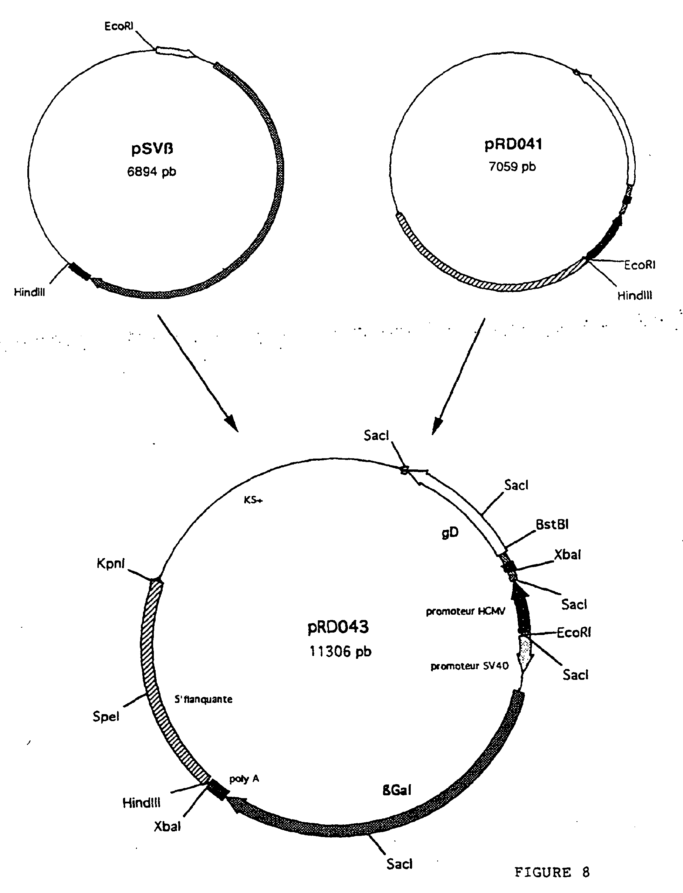

Le plasmide pSVβ (CLONTECH Laboratories, Palo Alto, CA) a été digéré par EcoRI et HindIII pour isoler le fragment EcoRI-HindIII de 4300 pb. Ce fragment a été cloné dans le plasmide pRD041 digéré par EcoRI et HindIII pour donner le plasmide pRD043 (11306 pb) (Figure 8) comprenant en plus par rapport au pRD041, le gène LacZ et son promoteur SV40.

Le plasmide pRD053 permet à la fois de remplacer le promoteur naturel de gD par le promoteur HCMV IE et d'introduire, par recombinaison homologue en utilisant le bras 5' correspondant au fragment allant des bases 2152 à 4245 (Zelnik 1993 - supra) (fragment correspondant à la plus grande partie du gène sORF-3, au gène US2 et au début du gène US3) et le bras 3' allant des positions 5747 (A de l'ATG du gène gD) à 6980 (ce qui correspond au gène gD et au polyA).

On comprend que, au lieu d'apporter le gène LacZ, on pourrait tout à fait aussi facilement apporter un gène codant pour un antigène d'intérêt.

### Exemple 3 : Construction du plasmide pRD053 (cassette CMV-gD dans TK)

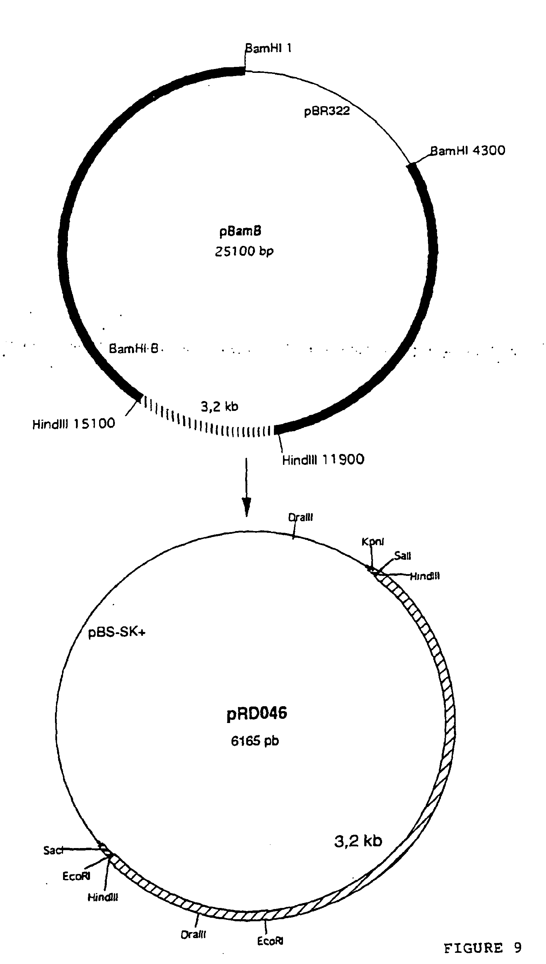

Le fragment HVT BamHI B (Igarashi T. et al., Virology. 1987, 157, 351-358) contenant le gène TK a été cloné dans le vecteur pBR322 pour donner le plasmide pBamB. Le plasmide pBamB a été digéré par HindIII pour isoler le fragment HindIII-HindIII de 3200 pb. Ce fragment a été cloné dans le vecteur pBS-SK+ digéré par HindIII pour donner le plasmide pRD046 (Figure 9). Le plasmide pRD046 a été digéré par SalI, réparé avec la polymérase Klenow puis digéré par SacI pour isoler le fragment SalI(K)-SacI de 3200 pb. Ce fragment a ensuite été ligaturé avec le vecteur pUC18 préalablement digéré par HindII et SacI pour donner le plasmide pRD049 (5943 pb). Le plasmide pRD049 a alors été digéré par DraIII (site unique sur pRD049), déphosphorylé par l'action de la phosphatase alcaline, et ligaturé avec un oligonucléotide synthétique double brin obtenu par hybridation des deux oligonucléotides suivants :
RD053 SEQ ID No 5 : 5' GTGGTACCATAGTCGACACCCT 3'
RDO54 SEQ ID No 6 : 5' GTGTCGACTATGGTACCACAGG 3'

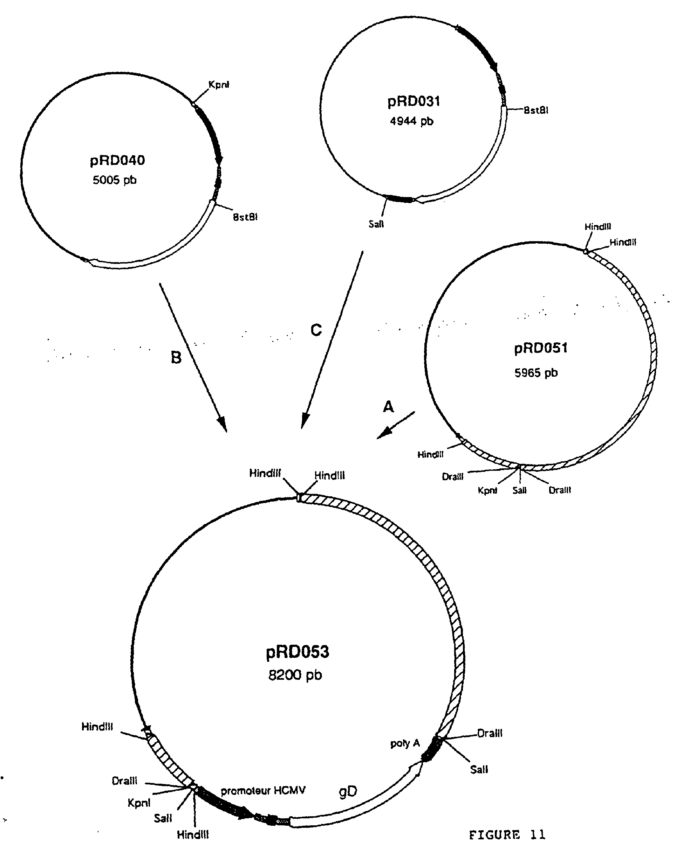

Le plasmide ainsi obtenu a été appelé pRD051 (Figure 9). Ce plasmide a été digéré par KpnI et SalI pour isoler le fragment KpnI-SalI de 5960 pb (fragment A). Le plasmide pRD040 (voir exemple 1) a été digéré par KpnI et BstBI pour isoler le fragment KpnI-HCMV IE- ATG-BstBI de 800 pb (fragment B). Le plasmide pRD031 a été digéré par BstBI et SalI pour isoler le fragment BstBI-HVT gD-SalI de 1450 pb (fragment C). Les fragments A, B et C ont ensuite été ligaturés ensemble pour donner le plasmide pRD053 (Figure 11) comprenant la cassette de gD insérée dans TK.

Ce plasmide permet l'introduction de cette cassette gD dans le gène TK par recombinaison homologue en utilisant des bras flanquants 5' et 3' respectivement homologues des régions en amont du site DraIII du gène TK (fragment HindIII-DraIII de 520 pb) et en aval du site DraIII du gène TK (fragment DraIII-HindIII de 2650 pb). Cette insertion conduit à l'inactivation de TK.

Ce plasmide est linéarisé par ScaI avant la cotransfection avec l'ADN viral d'HVT.

### Exemple 4 : Isolement et purification du virus recombinant vHVT004

L'ADN viral utilisé pour les expériences de transfection a été préparé selon la technique décrite par Robin Morgan (Morgan R. W. et al., Avian Diseases, 1990, 34, 345-351 incorporé ici par référence). Le plasmide pRD043 a été digéré par KpnI pour linéarisation, puis extrait avec un mélange phénol/chloroforme (19:1), précipité avec de l'éthanol absolu, et repris dans de l'eau stérile.

Des cellules CEPs primaires de 24 heures ont été transfectées avec le mélange suivant: 5 µg d'ADN viral+ 1µg du plasmide pRD043 linéarisé dans 300 µl de milieu OptiMEM (Gibco) et 100 µg de Lipofectamine (Gibco BRL Ref. 18324-012) dans 300 µl de milieu (volume final du mélange = 600 µl). Ces 600 µl ont été ensuite dilués dans 3 ml (volume final) de milieu et étalés sur 3.10⁶ CEPs. Le mélange est laissé 5 heures en contact avec les cellules, puis éliminé et remplacé par 5 ml de milieu de culture. Les cellules sont alors laissées en culture 3 jours à 37° C, puis elles sont pronasées, mélangées à des CEPs secondaires fraîches (mélange 3:1), et réétalées sur boîtes de Petri jusqu'à apparition d'un effet cytopathique (ECP). Le tapis cellulaire est alors pronasé et sert d'inoculum (à raison de 1 plage par boîte) pour des boîtes de 60 mm (contenant 2.10⁶ CEPs secondaires). Trois jours après l'inoculation, une couche d'agar est coulée sur le tapis cellulaire. 24 heures plus tard, une surcouche d'agar contenant de l'X-Gal (500 µg par ml final) est coulée. Trois heures après, une coloration bleue intense se développe au niveau des plages contenant les virus recombinants. Chaque plage bleue est alors repiquée individuellement pour amplification dans une cupule d'une plaque 96 puits. A l'apparition d'un ECP, la cupule est pronasée et sert d'inoculum pour une nouvelle boîte 60 mm. La sélection des plages recombinantes et l'estimation du pourcentage de purification de ces plaques se font comme précédemment par coloration X-Gal des plages sous agar. En général, 4 cycles d'isolements successifs suffisent pour obtenir des virus recombinants dont la totalité de la progénie présente la coloration bleue. Chaque plage individuelle purifiée à 100 % est alors caractérisée au niveau moléculaire par les techniques PCR et de Southern blot en utilisant les oligonucléotides et les sondes d'ADN appropriés.

Un clone pur à 100% et présentant les caractères du recombinant attendu a été isolé et désigné vHVT004. Ce virus contient une cassette SV40-LacZ insérée entre les gènes US2 et gD ainsi que le promoteur HCMV IE placé directement en amont du gène gD et contrôlant ainsi ce gène dans son site d'origine. Ce virus est donc délété de US3, qui est le site d'insertion.

### Exemple 5 : Isolement et purification du virus recombinant vHVT005 (TK-)

L'ADN génomique du virus HVT utilisé pour les expériences de transfection a été préparé comme précédemment (Morgan R. et al., Avian Diseases, 1990, 34, 345-351). Les transfections ont été réalisées avec le plasmide pRD053 linéarisé par ScaI comme décrit dans l'exemple 3. Après 3 jours de culture, les cellules sont pronasées, mélangées à des CEPs secondaires fraîches et réétalées sur boîtes de Petri. A l'apparition d'un ECP, le tapis cellulaire est pronasé, et sert d'inoculum pour des boites de Petri de 60 mm. La culture est alors effectuée avec un milieu de culture contenant de la 1-β-D-arabinofuranosyl-thymine (ara T) (Sigma) à la concentration finale de 100 µg/ml de milieu de culture. L'ara T inhibe la réplication des virus HVT TK+ et seuls les mutants naturels TK- ou les recombinants TK- peuvent se répliquer en présence d'ara T. Trois jours après l'infection, une couche d'agar est coulée sur le tapis cellulaire. 24 heures après, les plages virales présentes sur le tapis cellulaire sont repiquées individuellement dans des cupules d'une plaque 96 puits. La culture de ces plages se fait en présence d'ara T dans le milieu de culture. A l'apparition d'un ECP, les cupules sont pronasées, des dilutions des cupules infectées sont étalées sur des boîtes de Petri, et un nouveau cycle de culture sous agar est effectué comme précédemment. Après deux cycles de culture en présence d'ara T, la culture peut être effectuée en milieu normal. Quatre cycles d'isolement/purification sont suffisants pour obtenir des clones recombinants purs à 100 % pour le caractère TK-. Ces clones sont alors caractérisés au niveau moléculaire avec les techniques habituelles de la biologie moléculaire (PCR, Southern blot, etc...).

Un clone pur à 100% et présentant les caractères du recombinant attendu a été isolé et désigné vHVT005. Ce virus contient le gène gD sous le contrôle du promoteur HCMV IE dans le gène TK, lequel est donc inactivé.

## Revendications

1. Un virus de la maladie de Marek (MDV) de sérotype 1, 2 ou 3 recombiné (i) dans lequel le gène gD du virus MDV est placé sous le contrôle d'un promoteur autre que le promoteur naturel de ce gène et apte à induire l'expression du gène gD lors de la réplication du virus en culture cellulaire *in vitro,* ou (ii) qui comprend une cassette d'expression comportant un gène gD placé sous le contrôle d'un promoteur autre que le promoteur naturel de ce gène et permettant son expression lors de la réplication du virus en culture cellulaire *in vitro,* la cassette étant insérée dans le génome du MDV, dans un site non-essentiel pour la réplication du virus et pour l'efficacité du virus à titre du vaccin.

2. Le virus MDV selon la revendication 1, dans lequel le site d'insertion est choisi parmi le groupe consistant en : US2, US3, US10, gI, gD, TK, UL13, RR2.

3. Le virus MDV selon l'une quelconque des revendications 1 à 2, dans lequel le promoteur intégré pour assurer l'expression *in vitro* d'un gène gD est le promoteur HCMV IE ou le promoteur SV40.

4. Le virus MDV selon l'une quelconque des revendications 1 à 3, comprenant en outre au moins un gène hétérologue codant pour un antigène d'intérêt, inséré dans le génome du MDV de manière que ce gène puisse s'exprimer *in vivo.*

5. Le virus MDV selon l'une quelconque des revendications de 1 à 3, dans lequel la cassette d'expression selon la revendication 1 comprend en outre au moins un gène hétérologue codant pour un antigène d'intérêt placé sous le control d'un promoteur approprié.

6. Vaccin comprenant un virus MDV recombiné selon l'une quelconque des revendications 1 à 5.

7. Vaccin selon la revendication 6, utilisable par les voies parentérale, orale ou aérienne.

8. Vaccin selon la revendication 6 ou 7, sous forme lyophilisée ou congelée.

9. Procédé de culture d'herpèsvirus, de virus MDV de sérotype 1, 2 ou 3, dans lequel on cultive sur cellules le virus MDV selon l'une quelconque des revendications 1 à 5, puis l'on recueille les virions produits dans le surnageant de culture.

10. Utilisation d'un virus MDV recombiné selon l'une quelconque des revendications 1 à 5, pour la fabrication d'un vaccin contre la maladie de Marek, destiné à être administré à un oiseau d'élevage par voie parentérale, aérienne ou orale.

11. Utilisation selon la revendication 10, la voie aérienne étant la voie nasale ou la voie oculaire.

## Claims

1. A virus of Marek's disease (MDV) of serotype 1, 2 or 3 which is recombined (i) in which the gD gene of the MDV virus is placed under the control of a promoter other than the natural promoter of that gene and capable of inducing expression of the gD gene upon replication of the virus in in vitro cellular culture, or (ii) which comprises an expression cassette comprising a gD gene placed under the control of a promoter other than the natural promoter of that gene and permitting expression thereof upon replication of the virus in in vitro cellular culture, the cassette being inserted into the genome of the MDV in a site which is non-essential for replication of the virus and for the efficacy of the virus as a vaccine.

2. The MDV virus according to claim 1 wherein the insertion site is selected from the group consisting of: US2, US3, US10, gI, gD, TK, UL13 and RR2.

3. The MDV virus according to either one of claims 1 and 2 wherein the promoter which is integrated to ensure in vitro expression of a gD gene is the HCMV IE promoter or the SV40 promoter.

4. The MDV virus according to any one of claims 1 to 3 further comprising at least one heterologous gene coding for an antigen of interest, which is inserted into the genome of the MDV in such a way that said gene can be expressed in vivo.

5. The MDV virus according to any one of claims 1 to 3 wherein the expression cassette according to claim 1 further comprises at least one heterologous gene coding for an antigen of interest which is placed under the control of a suitable promoter.

6. A vaccine comprising a recombined MDV virus according to any one of claims 1 to 5.

7. A vaccine according to claim 6 which can be used by way of parenteral, oral or air routes.

8. A vaccine according to claim 6 or claim 7 which is in lyophilised or frozen form.

9. A process for culturing herpesvirus, MDV virus of serotype 1, 2 or 3, wherein the MDV virus according to any one of claims 1 to 5 is cultured on cells and then the virions produced are collected in the culture supernatant.

10. Use of a recombined MDV virus according to any one of claims 1 to 5 for the production of a vaccine against Marek's disease, intended to be administered to a breeding bird by a parenteral, air or oral route.

11. Use according to claim 10, the air route being the nasal route or the ocular route.

## Patentansprüche

1. Rekombinanter Virus der Marekschen Krankheit (MDV) vom Serotyp 1, 2 oder 3,
(i) bei dem das gD-Gen des MDV-Virus unter der Kontrolle eines Promotors platziert ist, der ein anderer ist als der natürliche Promotor dieses Gens und in der Lage ist, die Expression des gD-Gens bei der Replikation des Virus in Zellkultur *in vitro* zu induzieren, oder
(ii) der eine Expressionskassette umfasst, die ein gD-Gen enthält, das unter der Kontrolle eines Promotors platziert ist, der ein anderer ist als der natürliche Promotor dieses Gens, und dessen Expression bei der Replikation des Virus in Zellkultur *in vitro* ermöglicht, wobei die Kassette im Genom des MDV in einer Stelle inseriert wurde, die nicht essentiell für die Replikation des Virus und für die Wirksamkeit des Virus als Vakzin ist.

2. MDV-Virus nach Anspruch 1, bei dem die Insertionsstelle ausgewählt ist aus der Gruppe bestehend aus: US2, US3, US10, gI, gD, TK, UL13, RR2.

3. MDV-Virus nach einem der Ansprüche 1 bis 2, bei dem der Promotor, der integriert ist, um die *in vitro* Expression eines gD-Gens sicherzustellen, der HCMV IE- oder der SV40-Promotor ist.

4. MDV-Virus nach einem der Ansprüche 1 bis 3, der weiterhin mindestens ein heterologes Gen umfasst, das ein Antigen von Interesse codiert, welches im MDV-Genom in der Weise inseriert ist, dass das Gen *in vivo* exprimiert werden kann.

5. MDV-Virus nach einem der Ansprüche 1 bis 3, bei dem die Expressionskassette nach Anspruch 1 weiterhin mindestens ein heterologes Gen umfasst, das ein Antigen von Interesse codiert, das unter der Kontrolle eines geeigneten Promotors platziert ist.

6. Vakzin, das einen rekombinanten MDV-Virus nach einem der Ansprüche 1 bis 5 umfasst.

7. Vakzin nach Anspruch 6, das über parenteralen-, oralen- oder Luftweg verwendbar ist.

8. Vakzin nach Anspruch 6 oder 7 in lyophilisierter oder gefrorener Form.

9. Verfahren zur Züchtung von Herpesvirus, von MDV-Virus vom Serotyp 1, 2 oder 3, bei dem der MDV-Virus nach einem der Ansprüche 1 bis 5 auf Zellen kultiviert wird, worauf die Virionen, die in dem Kulturüberstand produziert werden, geerntet werden.

10. Verwendung eines rekombinanten MDV-Virus nach einem der Ansprüche 1 bis 5 zur Herstellung eines Vakzins gegen die Mareksche Krankheit, das zur Verabreichung an Zuchtvögel über parenteralen-, Luft- oder oralen Weg bestimmt ist.

11. Verwendung nach Anspruch 10, wobei der Luftweg der nasale oder okulare Weg ist.
